# EUROPEAN PATENT APPLICATION

(11) **EP 4 176 876 A1**
(43) Date of publication of application: **10.05.2023**
(21) Application number: 22199365.2
(22) Date of filing: 14.09.2018
(51) Int. Cl.: A61K 31/405, A61P 35/00

(54) **METHODS AND COMPOSITIONS FOR THE TREATMENT OF CANCER**

(30) Priority: 14.09.2017 US 201762558573 P
(62) Divisional of application: 18856836.4
(71) Applicant: Lankenau Institute for Medical Research, Wynnewood, PA 19096 (US)
(72) Inventor: MULLER, Alexander J, Swarthmore, PA 19082 (US); MONDAL, Arpita, Havertown, PA 19083 (US); DEY, Souvik, Wynnewood, PA 19090 (US); PRENDERGAST, George, C., Penn Valley, PA 19072 (US)
(74) Representative: Appleyard Lees IP LLP

(57) **Abstract**

Compositions and methods for the treatment of cancer are disclosed.

## Description

### FIELD OF THE INVENTION

This invention relates to the field of chemotherapy. Specifically, the invention provides novel compositions and methods for the treatment of cancer.

### BACKGROUND OF THE INVENTION

Neovascularization is critical for tumor outgrowth (Cao et al. (2011) Sci. Transl. Med., 3:114rv3). However, unlike the tightly regulated process of normal tissue vascularization, tumor neovascularization is characterized by excessive and disorganized growth of blood vessels much like that induced by ischemia in tissues such as the retina and lungs (Carmeliet, P. (2003) Nat. Med., 9:653-660). An emergent concept in the field of angiogenesis is that normalizing pathologic tumor vasculature will enhance the efficacy of chemo- and radiotherapies. Accordingly, new synergistic combinations of agents which exploit the pathogenic neovascularization of tumors are sought.

### SUMMARY OF THE INVENTION

In accordance with one aspect of the instant invention, methods for treating, inhibiting (e.g., reducing), and/or preventing a cancer in a subject are provided. The methods comprise administering at least one inhibitor of the induction or activity of tryptophan degradation and/or of the downstream pathways that respond to this process; and administering at least one second agent, wherein the second agent is an anti-vasculogenic/anti-angiogenic agent and/or therapeutic agent which acts by imposing nutrient/oxygen starvation on a cancer cell or exploiting nutrient/oxygen starvation in a cancer cell. In a particular embodiment, the methods comprise the administration of an inhibitor of indoleamine 2,3-dioxygenase-1 (IDO1) with said second agent(s). The combination of administered agents acts synergistically to inhibit, treat, and/or prevent the cancer in the subject, compared to the activity of the agents individually. In a particular embodiment, the IDO1 signaling inhibitor is a small molecule inhibitor (e.g., 1-methyl-tryptophan).

In accordance with another aspect of the instant invention, compositions for treating, inhibiting, and/or preventing cancer in a subject are also provided.

Aspects of the invention will now be detailed with reference to the following paragraphs:
Paragraph 1. A method of treating, inhibiting, and/or preventing a cancer in a subject comprising
   1) administering to said subject at least one inhibitor of induction, activity, or signaling by indoleamine 2,3 dioxygenase-1 (IDO1); and
   2) administering to said subject at least one second agent, wherein said second agent is an anti-vasculogenic/anti-angiogenic agent and/or therapeutic agent which acts by imposing nutrient/oxygen starvation on a cancer cell or exploiting nutrient/oxygen starvation in a cancer cell.
Paragraph 2. The method of paragraph 1, wherein said cancer is lung cancer.
Paragraph 3. The method of paragraph 1, wherein said cancer comprises pulmonary metastasis.
Paragraph 4. The method of paragraph 1, wherein said inhibitor is a direct IDO1 inhibitor.
Paragraph 5. The method of paragraph 4, wherein said IDO1 inhibitor is a small molecule inhibitor.
Paragraph 6. The method of paragraph 5, wherein said IDO1 inhibitor is 1-methyl-tryptophan.
Paragraph 7. The method of paragraph 1, wherein said second agent is selected from the group consisting of an anti-vasculogenic/anti-angiogenic agent, a hypoxia-activated prodrug or bioreductive drug, a molecularly targeted drug which exploits responses to hypoxia, and a molecularly targeted drug which exploits responses to nutrient or oxygen deprivation induced stress.
Paragraph 8. The method of paragraph 7, wherein said hypoxia-activated prodrug or bioreductive drug is selected from the group consisting of PR-104, evofosfamide, and tarloxotinib.
Paragraph 9. The method of paragraph 7, wherein said molecularly targeted drugs which exploit responses to hypoxia are HIF-1α inhibitors.
Paragraph 10. The method of paragraph 7, wherein said second agent is 2-mercaptopropionyl glycine disulfide.
Paragraph 11. A composition comprising at least one inhibitor of indoleamine 2,3 dioxygenase-1 (IDO1) signaling; an anti-vasculogenic/anti-angiogenic agent and/or therapeutic agent which acts by imposing nutrient/oxygen starvation on a cancer cell or exploiting nutrient/oxygen starvation in a cancer cell; and at least one pharmaceutically acceptable carrier.

### BRIEF DESCRIPTION OF THE DRAWINGS

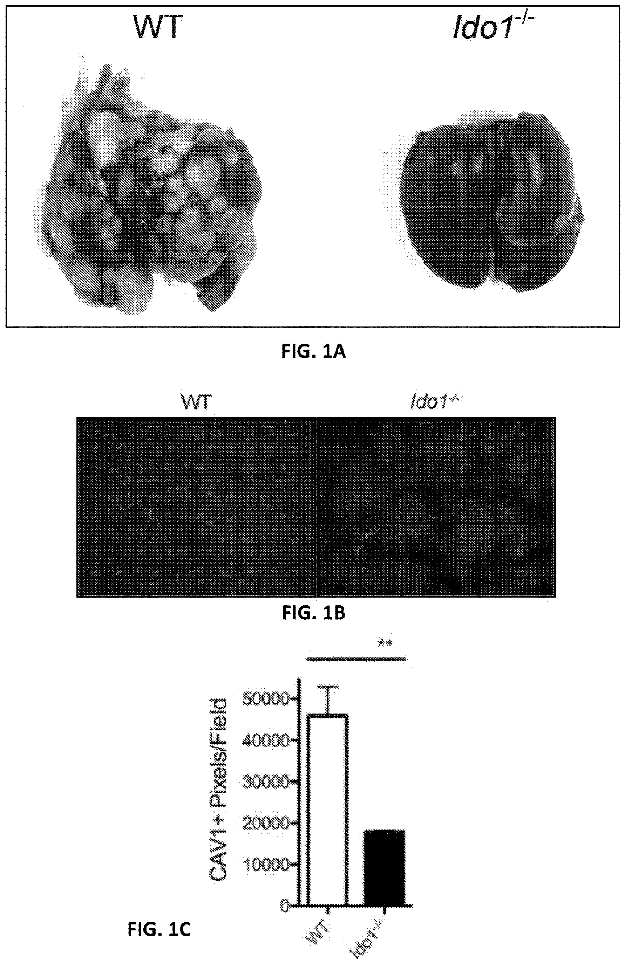

Figure 1A provides images of the differential outgrowth of 4T1 pulmonary metastases in mice with or without IDO1. Images of lungs stained with India ink to visualize the metastatic burden in BALB/c WT and *Ido1*^{*-*/*-*} mice at 5 weeks following orthotopic 4T1 mammary tumor cell engraftment are provided. Figure 1B provides representative images of immunofluorescent staining of blood vessels with anti-Cav1 and of nuclei with DAPI within 4T1 lung metastases in WT and *Ido1*^{*-*/*-*} mice. Figure 1C provides a quantitative assessment of neovascular density as marked by anti-Cav1 positive staining within 4T1 lung metastases in WT and *Ido1*^{*-*/*-*} mice (N ≥ 3 mice). Data are graphed as means ± SEM with significance determined by 2-tailed Student's t-test.

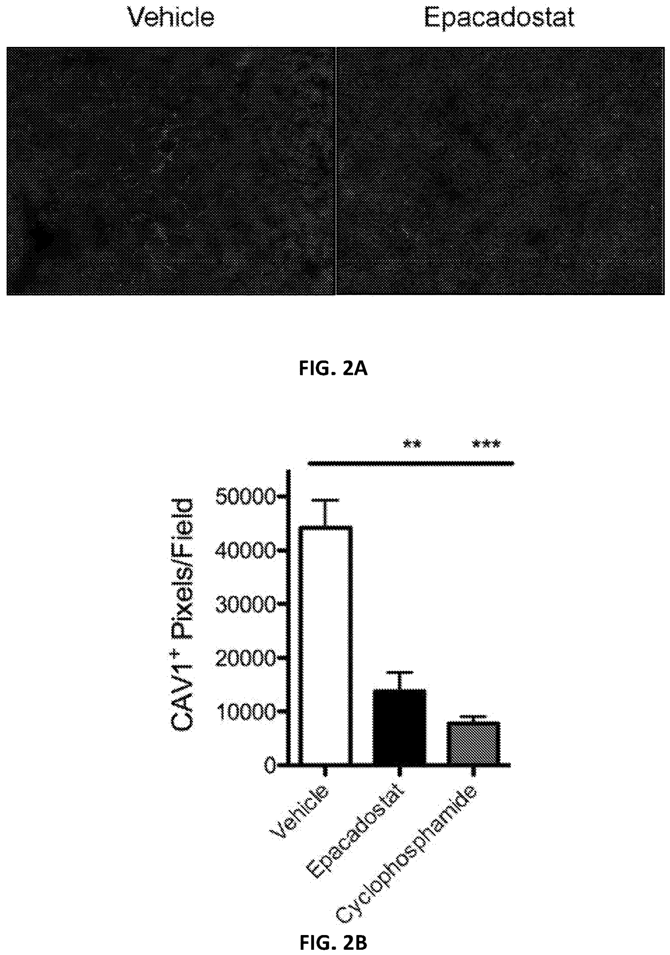

Figure 2A provides representative images of immunofluorescent staining of blood vessels with anti-Cav1 and of nuclei with DAPI within metastatic regions of lung specimens prepared from WT mice dosed orally (p.o.) with vehicle or 50 mg/kg epacadostat, b.i.d., over 3 days beginning 3.5 weeks after orthotopic 4T1 mammary tumor cell engraftment. Figure 2B provides a quantitative assessment of neovascular density marked by anti-Cav1 positive staining within lung metastases of WT mice administered either vehicle or epacadostat over 3 days together with a third positive control cohort that received a single i.p. injection of 50 mg/kg cyclophosphamide at 3.5 weeks after orthotopic 4T1 mammary tumor cell engraftment for evaluation 3 days later (N ≥ 3 mice). Data are graphed as means ± SEM with significance determined by one-way ANOVA with Dunnett's multiple comparison test.

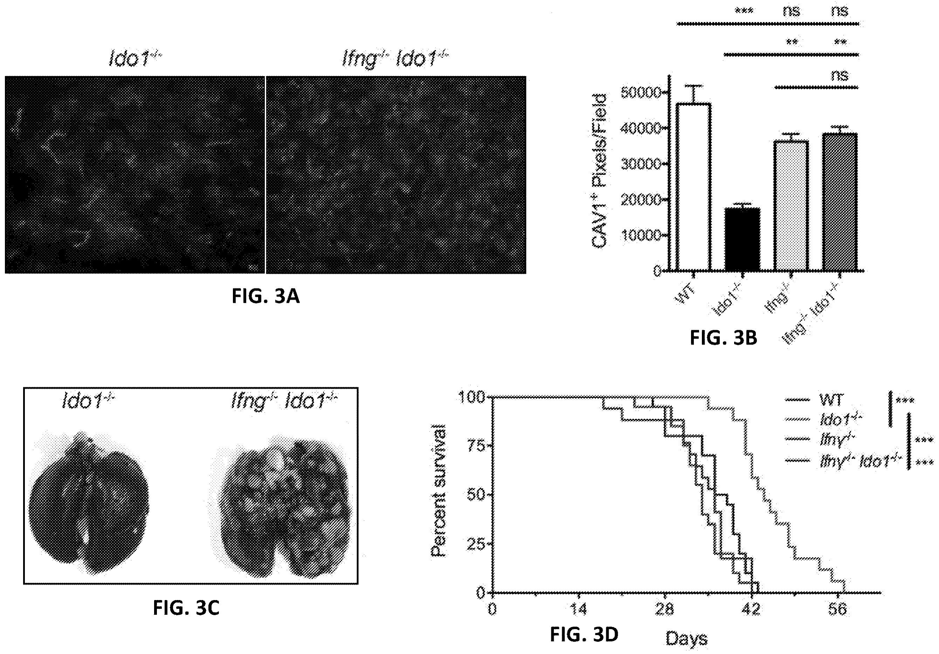

Figure 3A provides representative images of immunofluorescent staining of blood vessels with anti-Cav1 and of nuclei with DAPI within metastatic regions of lung specimens prepared from *Ido1*^{*-*/*-*} and *Ifng*^{*-*/}*⁻ Ido1*^{*-*/*-*} mice following orthotopic 4T1 mammary tumor cell engraftment. Figure 3B provides a quantitative assessment of neovascular density marked by anti-Cav1 positive staining within lung metastases of WT, *Ido1*^{*-*/}*⁻ Ifng*^{*-*/*-*} and *Ifng*^{*-*/}*⁻ Ido1*^{*-*/*-*} mice following orthotopic 4T1 mammary tumor cell engraftment (N ≥ 3 mice). The data are graphed as means ± SEM with significance determined by one-way ANOVA with Tukey's multiple comparison test. Figure 3C provides images of the staining of lungs with India ink to visualize the metastatic burden in *Ido1*^{*-*/*-*} and *Ifng*^{*-*/}*⁻ Ido1*^{*-*/*-*} mice at 5 weeks following orthotopic 4T1 mammary tumor cell engraftment. Figure 3D provides Kaplan-Meier survival curves for cohorts of WT, *Ido1*^{*-*/*-*}, *Ifng*^{*-*/*-*} and *Ifng*^{*-*/}*⁻ Ido1*^{*-*/*-*} mice following orthotopic engraftment of 1 × 10⁴ 4T1 cells (N ≥ 17 mice) with significance assessed by 2-group log-rank test.

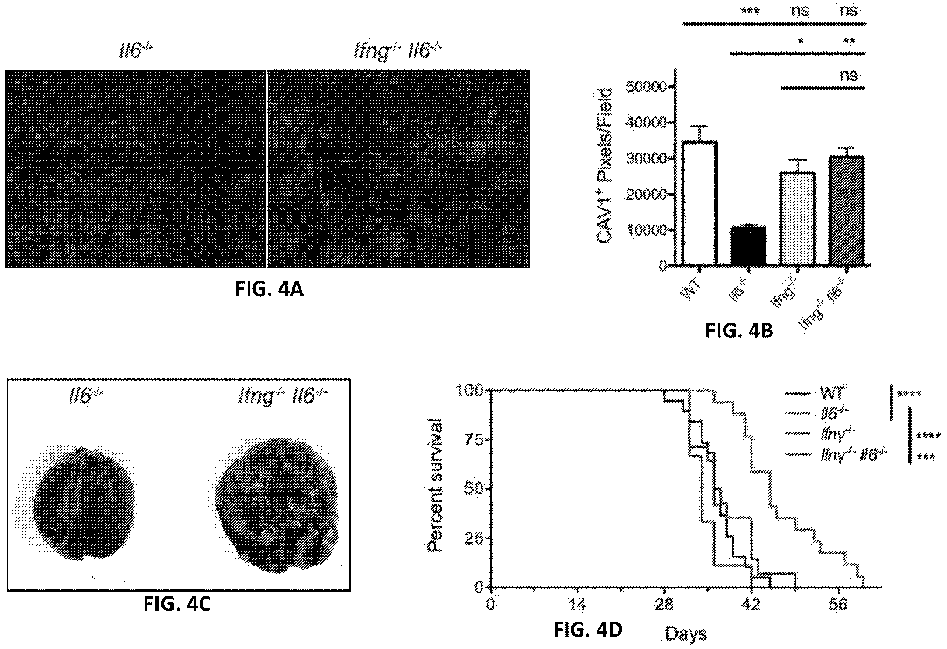

Figure 4A provides representative images of immunofluorescent staining of blood vessels with anti-Cav1 and of nuclei with DAPI within metastatic regions of lung specimens prepared from WT, *Il6*^{*-*/*-*}, *Ifng*^{*-*/*-*} and *Ifng*^{*-*/}*⁻ Il6*^{*-*/*-*} mice following orthotopic 4T1 mammary tumor cell engraftment. Figure 4B provides a quantitative assessment of neovascular density marked by anti-Cav1 positive staining within lung metastases of WT, *Il6*^{*-*/*-*}, *Ifng*^{*-*/*-*} and *Ifng*^{*-*/}*⁻ Il6*^{*-*/*-*} mice following orthotopic 4T1 mammary tumor cell engraftment (N ≥ 3 mice). The data are graphed as means ± SEM with significance determined by one-way ANOVA with Tukey's multiple comparison test. Figure 4C provides images of the staining of lungs with India ink to visualize the metastatic burden in *Il6*^{*-*/*-*} and *Ifng*^{*-*/}*⁻ Il6*^{*-*/*-*} mice at 5 weeks following orthotopic 4T1 mammary tumor cell engraftment. Figure 4D provides Kaplan-Meier survival curves for cohorts of WT, *Il6*^{*-*/}*⁻, Ifng*^{*-*/*-*} and *Ifng*^{*-*/}*⁻ Il6*^{*-*/*-*} mice following orthotopic engraftment of 1 × 10⁴ 4T1 cells (N ≥ 9 mice) with significance assessed by 2-group log-rank test.

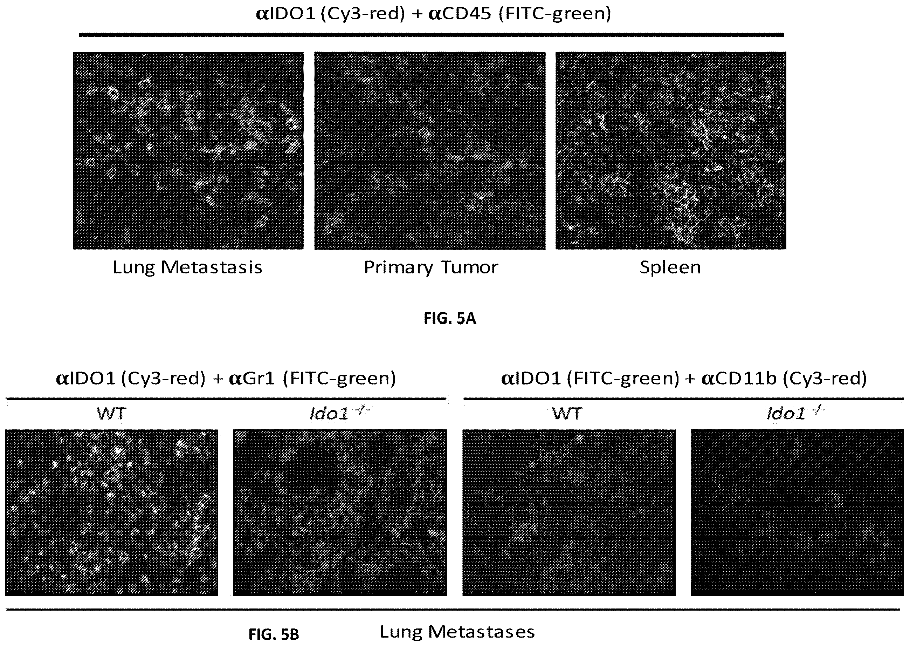

Figure 5A provides images of immunofluorescence staining of metastatic lung nodules and spleen with anti-IDO1 and anti-CD45 antibodies. Figure 5B provides images of immunofluorescence staining of metastatic lung nodules with anti-IDO1 and anti-Gr1 antibodies or anti-IDO1 and anti-CD11b antibodies.

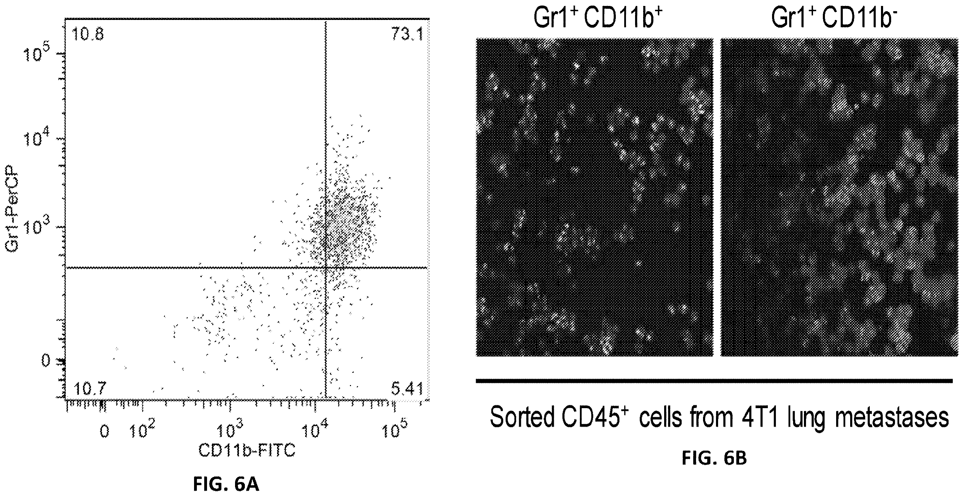

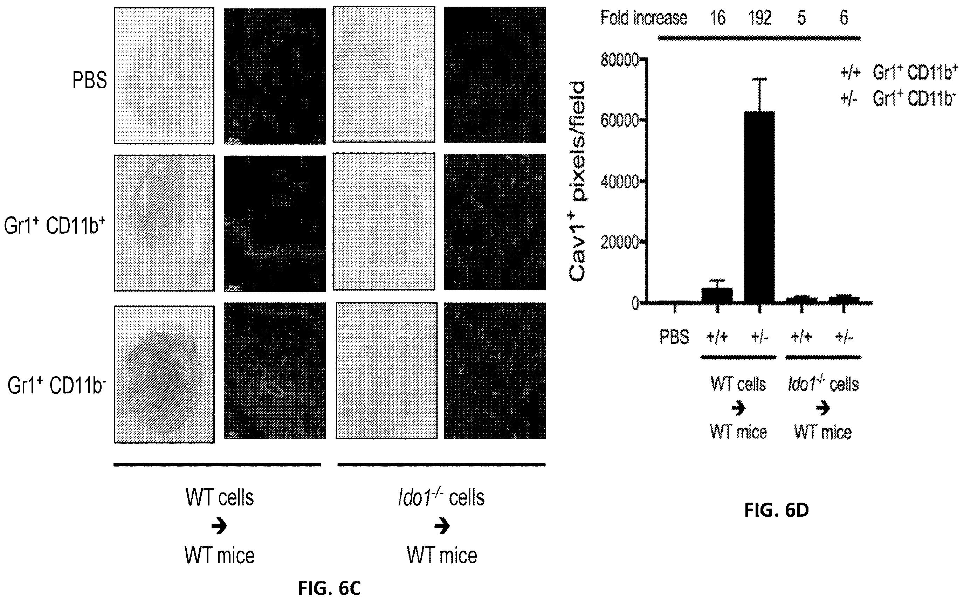

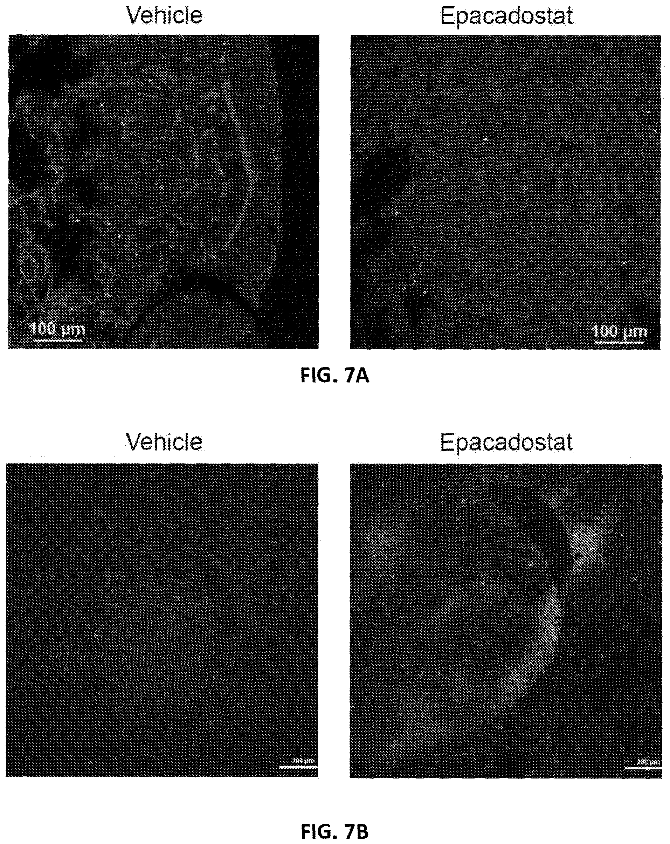

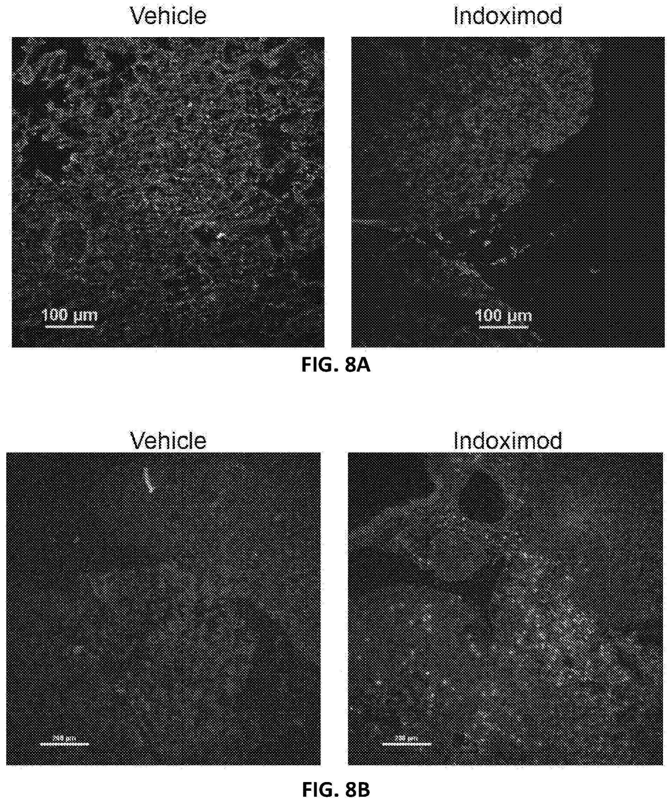

Figure 6A provides flow cytometry data on immune cells isolated from 4T1 lung metastases. Figure 6B provides fluorescence microscopy images of Gr1⁺ CD11b⁺ and Gr1⁺ CD11b⁻ sorted cell populations with anti-IDO1 and anti-CD11b antibodies. Figure 6C provides images of resected Matrigel plugs with sorted Gr1⁺ CD11b⁺ or Gr1⁺ CD11b⁻ cells that were engrafted into mice and fluorescence microscopy images of infiltrating neovascularization in the resected plugs stained with anti-caveolin-1 antibody. Figure 6D provides a quantitation of the vascular density in the resected Matrigel plugs.

### DETAILED DESCRIPTION OF THE INVENTION

Chronic inflammation provides a microenvironmental context which can foster tumor promotion through a complex and dynamic interplay between stroma and tumor that remains an area of active investigation (Peek et al. (2005) Cancer Res., 65:8583-8586). In an experimental model of two stage chemical carcinogenesis, wherein mutagenic tumor initiation and inflammatory tumor promotion are distinctly separable, IDO1 (indoleamine 2,3-dioxygenase 1) has been identified as a vital component of the inflammatory tumor promoting milieu (Muller et al. (2008) Proc. Natl. Acad. Sci., 105:17073-17078). Enzymatically, IDO1 catabolizes the essential amino acid tryptophan, but it is not the enzyme responsible for maintaining normal tryptophan homeostasis, which instead is the role of the evolutionarily convergent liver enzyme TDO2 (tryptophan dioxygenase 2). Rather, IDO1 can be expressed in a variety of tissues, particularly along mucosal surfaces, and is strongly induced by the inflammatory cytokine IFNγ (interferon-γ) (Taylor et al. (1991) FASEB J., 5:2516-2522). The conceptualization of IDO1 as a regulator of immune function emerged from observations that tryptophan depletion by IDO1 could suppress cytotoxic T cell activation (Munn et al., 1999). The demonstration that an IDO1 pathway inhibitor 1MT (1-methyl-tryptophan) could elicit T cell-dependent rejection of allogeneic mouse concepti (Munn et al. (1998) Science 281:1191-1193) dramatically cemented the concept of IDO1 as a tolerogenic actor. Subsequent findings linking loss of the tumor suppressor gene Bin1 to IDO1 dysregulation and tumoral immune escape (Muller et al. (2005) Nat. Med., 11:312-319) provided experimental substantiation for the corollary proposition that tumors might appropriate this mechanism for protecting the 'foreign' fetus to overcome tumor immunosurveillance. Additionally, while IDO1 can contribute to tumor development when expressed directly within tumor cells, (where immunoediting may be at play), its expression within non-malignant stroma has been shown to be contributory to tumor development as well (Munn et al. (2004) J. Clin. Invest., 114:280-290).

The development of tumors in the lungs has been studied as this is an organ in which the constitutive level of IDO1 is relatively high (Takikawa et al. (1986) J. Biol. Chem., 261:3648-3653). Genetic loss of IDO1 resulted in a marked decrease in the pulmonary tumor burden in both a transgenic mouse model of de novo lung carcinoma as well as in an orthotopic graft model of metastatic breast cancer. In both instances this translated to a significant survival benefit in the *Ido1*^{*-*/*-*} animals (Smith et al. (2012) Cancer Discov., 2:722-735).

As explained hereinabove, angiogenesis is critical to tumor development (Cao et al. (2011) Sci. Transl. Med., 3(114):114rv113). Unlike physiologic angiogenesis which is tightly regulated, in cancer there is excessive and disorganized growth of blood vessels much like that induced by ischemia in tissues such as the retina and lungs. In experimental models of ischemia, immune cells have been shown to be important for pruning the excess vasculature and limiting neovascularization (Ishida et al. (2003) Nat. Med., 9:781-788; Wagner et al. (2008) Am. J. Physiol. Lung Cell. Mol. Physiol., 294:L351-357). Thus, immunity might play an important anti-angiogenic role in tumors as well.

One important check on tumor neovascularization is the inflammatory cytokine IFNγ. The loss of tumor vasculature elicited by IFNγ, rather than direct tumor cell killing, has been implicated as being the primary mechanism for both CD4+ and CD8+ T cell-mediated tumor rejection (Qin et al. (2000) Immunity 12:677-686; Qin et al. (2003) Cancer Res., 63:4095-4100). The inflammatory cytokine IFNγ is a major inducer of IDO1 (Yoshida et al. (1981) Proc. Natl. Acad. Sci., 78:129-132). Thus, IDO1 may act in a negative feedback capacity to dampen the tumor suppressive, antineovascular effects of IFNγ. Notably, IDO1 can potentiate the induction of the inflammatory cytokine IL6 (interleukin 6) (Smith et al. (2012) Cancer Discov., 2:722-735), which has been implicated as a pro-neovascular factor for tumors (Middleton et al. (2014) Crit. Rev. Oncol. Hematol., 89:129-139; McClintock et al. (2005) J. Appl. Physiol., 99:861-866). Herein, it is demonstrated that IDO1 has a critical role in supporting neovascularization that corresponds with its integration at the interface between these two competing inflammatory cytokines, IFNγ and IL6.

The present invention provides compositions and methods for the inhibition (e.g., reduction, slowing, etc.), prevention, and/or treatment of cancer. The present invention also provides compositions and methods for the inhibition, prevention, and/or treatment of pathogenic neovascularization. The methods comprise administering at least one inhibitor of the induction or activity of tryptophan degradation and/or of the downstream pathways that respond to this process and at least one anti-vasculogenic/anti-angiogenic agent and/or therapeutic agent which acts by imposing nutrient/oxygen starvation on a target tissue (e.g., cancer cell or tumor) or exploiting nutrient/oxygen starvation in a target tissue ("therapeutic agent") to a subject (e.g., a subject in need thereof (e.g., a subject with cancer)). In a particular embodiment, the methods comprise the administration of an inhibitor of IDO1.

The agents administered to the subject may be contained with a single composition comprising at least one carrier (e.g., pharmaceutically acceptable carrier). Alternatively, the agents may be administered separately (e.g., administered in separate compositions comprising at least one carrier (e.g., pharmaceutically acceptable carrier)). In a particular embodiment, the agents may be administered sequentially and/or concurrently. For example, the IDO1 signaling inhibitor may be administered before, after, and/or at the same time as the anti-vasculogenic/anti-angiogenic agent and/or therapeutic agent. Similarly, the anti-vasculogenic/anti-angiogenic agent and/or therapeutic agent may be administered before, after, and/or at the same time as the IDO1 signaling inhibitor. When the agents are not administered at the same time, the agents should be administered close enough in time such that the agents are capable of acting synergistically in the patient.

As stated hereinabove, the methods comprise administering at least one inhibitor of the induction or activity of tryptophan degradation or of the downstream pathways that respond to this process to a subject. In a particular embodiment, the inhibitor is a small molecule inhibitor (e.g., a small molecule inhibitor of IDO1). In a particular embodiment, the inhibitor is an inhibitory nucleic acid molecule (e.g., antisense, siRNA, shRNA, etc.) or a vector encoding the same. In a particular embodiment, the inhibitor is an antibody or antibody fragment immunologically specific for the protein to be inhibited (e.g., a neutralizing antibody; e.g., an anti-IDO1 antibody or fragment thereof). In a particular embodiment, the IDO1 inhibitor is an IDO1-targeting, peptide-based vaccine (e.g., Iversen et al. (2014) Clin. Cancer Res., 20:221-32). In a particular embodiment, the IDO1 inhibitor does not substantially inhibit IDO2.

Examples of small molecule IDO1 inhibitors are provided, without limitation, in PCT/US2014/022680 (e.g., tricyclic compounds related to imidazoisoindoles; compounds of Formulas I-V), PCT/US2012/033245 (e.g., fused imidazole derivatives; compounds of Formula I or II), PCT/US2010/054289 (e.g., imidazole derivatives; compounds of Formulas I-VIII), PCT/US2009/041609 (e.g., compounds of Formulas I-VIII), PCT/US2008/57032 (e.g., napthoquinone derivatives; compounds of Formula I, II, or III), PCT/US2008/085167 (e.g., compounds of Formulas I-XLIV), PCT/US2006/42137 (e.g., compounds of Formula I), PCT/US2006/017983 (e.g., compounds of Formula I), PCT/US2004/005155 (e.g., phenyl-TH-DL-trp (3-(N-phenyl-thiohydantoin)-indole), propenyl-TH-DL-trp (3-(N-allyl-thiohydantoin)-indole), and methyl-TH-DL-trp (3- (N-methyl-thiohydantoin)-indole)), PCT/US2004/005154 (e.g., compounds of Formula I or II), U.S. Patent 7,705,022 (e.g., compounds of Formula I), U.S. Patent 8,008,281 (e.g., phenyl-TH-DL-trp (3-(N-phenyl-thiohydantoin)-indole), propenyl-TH-DL-trp (3-(N-allyl-thiohydantoin)-indole), and methyl-TH-DL-trp (3- (N-methyl-thiohydantoin)-indole)), U.S. Patent 7,714,139 (e.g., compounds of Formula I or II), U.S. Patent Application Publication No. 20140066625 (e.g., fused imidazole derivatives; compounds of Formula I or II), U.S. Patent Application Publication No. 20130177590 (e.g., N-hydroxyamidinoheterocycles; compounds of Formulas I-III), U.S. Patent Application Publication No. 20140023663 (e.g., 1,2,5-oxadiazoles; compounds of Formula I), U.S. Patent Application Publication No. 20080146624 (e.g., amidines; compounds of Formulas I or II), U.S. Patent Application Publication No. 20080119491 (e.g., amidinoheterocycles; compounds of Formulas I-IV), U.S. Patent Application Publication No. 20080182882 (e.g., N-hydroxyamidinoheterocycles; compounds of Formula I), U.S. Patent Application Publication No. 20080214546 (e.g., N-hydroxyamidinoheterocycles; compounds of Formula I), U.S. Patent Application Publication No. 20060258719 (compounds of Formula I), Banerjee et al. (2008) Oncogene 27:2851-2857 (e.g., brassinin derivatives; ), and Kumar et al. (2008) J. Med. Chem., 51:1706-1718 (e.g., phenyl-imidazole-derivatives). In a particular embodiment, the IDO1 inhibitor is a prodrug (see, e.g., U.S. Patent Application Publication No. 20170022157 and U.S. Provisional Application No. 62/555,726).

In a particular embodiment, the IDO1 inhibitor is epacadosat (INCB024360, Incyte; Wilmington, DE; Liu et al. (2010) Blood 115(17):3520-3530; Koblish et al. (2010) Mol. Cancer Ther., 9(2):489-498), navoximod (NLG919, GDC-0919, RG6078; NewLink Genetics/Genentech), BMS-986205 (F001287, Hunt et al., AACR 2017, Abstract 4964), PF-06840003 (Wythes et al, SITC 2016, Abstract 253), or betalapachone (3,4-dihydro-2,2-dimethyl-2H-naphthol[1,2-b]pyran-5,6-dione, Flick et al. (2013) Int. J. Tryp. Res. 6:35-45).

In a particular embodiment, the IDO1 induction inhibitor is ethyl pyruvate (Muller, et al. (2010) Cancer Res. 70:1845-1853) or gleevec (imatinib, Balachandran et al. (2011) Nat. Med. 17:1094-1100). In a particular embodiment, the IDO1 pathway inhibitor (e.g., inhibitor of downstream signaling pathway) is 1-methyl-tryptophan, particularly 1-methyl-D-tryptophan (indoximod, NLG-8189; 1-methyl-D-tryptophan; NewLink Genetics), including salts and prodrugs (U.S. Patent Application Publication No. 20170022157) or a racemic mix comprising the same.

Inhibitors of IDO1 expression include, without limitation, inhibitors of JAK/STAT (e.g., JAK, STAT3, STAT1) (Du et al. (2000) J. Interferon Cytokine Res., 20:133-142, Muller et al. (2005) Nature Med., 11:312-319; Yu et al. (2014) J. Immunol., 193:2574-2586) , NFκB (Muller et al. (2005) Nature Med., 11:312-319; Muller et al. (2010) Cancer Res., 70:1845-1853), KIT (Balachandran et al. (2011) Nature Med., 17:1094-1100), MET (Rutella et al. (2006) Blood 108:218-227; Giannoni et al. (2014) Haematologica 99:1078-1087), RAS/RAF/MEK (Liu (2010) Blood 115:3520-3530), aryl hydrocarbon receptor (AHR) (Bessede et al. (2014) Nature 511:184-190; Litzenburger et al. (2014) Oncotarget 5:1038-1051), or vascular endothelial growth factor receptor (VEGFR) (Marti et al. (2014) Mem Inst Oswaldo Cruz 109:70-79). In a particular embodiment, the inhibitor is not an inhibitor of VEGFR.

Inhibitors of IDO1 downstream signaling include, without limitation, inhibitors of GCN2 (Munn et al. (2005) Immunity 22:633-642; Muller (2008) Proc Nat Acad Sci., 105:17073-17078), C/EBP homologous protein 10 (CHOP-10; also known as gadd153; herein referred to as CHOP; Munn et al. (2005) Immunity 22:633-642), activating-transcription factor 4 (ATF4; Munn et al. (2005) Immunity 22:633-642; Thevenot et al. (2014) Immunity 41:389-401), or aryl hydrocarbon receptor (AHR) (Opitz et al. (2011) Nature 478:197-203; Litzenburger et al. (2014) Oncotarget 5:1038-1051); or activators of mammalian target of rapamycin (mTOR) or protein kinase C (PKC)-Θ (Metz et al. (2012) Oncoimmunology 1:1460-1468). In a particular embodiment, the inhibitor of IDO1 downstream signaling is an inhibitor of IL6 (e.g., an antibody immunologically specific for IL6).

In a particular embodiment, the agent administered with the IDO1 signaling inhibitor is an anti-vasculogenic/anti-angiogenic agent and/or therapeutic agent which acts by imposing nutrient/oxygen starvation (e.g., starvation or hypoxic state) or exploits nutrient/oxygen starvation in a target tissue. For example, the anti-vasculogenic/anti-angiogenic agent and/or therapeutic agent can be, without limitation: 1) an anti-vasculogenic/anti-angiogenic agent, 2) a hypoxia-activated prodrug or bioreductive drug, 3) a molecularly targeted drug which exploits responses to hypoxia (e.g., HIF-targeting drugs), or 4) a molecularly targeted drug which exploits responses to nutrient or oxygen deprivation induced stress (e.g., antimetabolites, UPR inhibitors).

In a particular embodiment, the anti-vasculogenic/anti-angiogenic agent is a chemotherapeutic agent with anti-angiogenic activity. In a particular embodiment, the anti-vasculogenic/anti-angiogenic agent is a VEGF antagonist capable of neutralizing, blocking, inhibiting, abrogating, reducing or interfering with VEGF activities including its binding to one or more VEGF receptor. For example, the VEGF antagonist can be, without limitation, an anti-VEGF antibody, a VEGF-trap, an anti-VEGFR antibody, a VEGFR inhibitor, thalidomide, a DI 14-Notch inhibitor, an anti-tubulin vascular disrupting agent (VDA), an angiopoietin-Tie2 inhibitor, a nitric oxide synthase (NOS) inhibitor, a cationic poly amino acid dendrimer, rapamycin, everolimus, temserolimus, a low molecular weight heparin, a SPARC (osteonectin) peptide, bevacizumab, ranibizumab, ramucirumab, aflibercept, interleukin 17 (IL-17), DC101, sunitinib, sorafenib, pazopanib, AMG706, cediranib, vandetanib, vargatef, brivanib, XL-184, axitinib, tivozanib, thalidomide, lanalidomide, DMXAA, nadroparin, 2,5-dimethyl-celecoxib, cyclophosphamide, HBC, and tasquinimod.

Examples of hypoxia-activated prodrugs/bioreductive drugs include, without limitation: PR-104 (Proacta; La Jolla, CA, Patterson, et al. (2007) Clin. Cancer Res. 13:3922-3932), evofosfamide (TH-302, Duan, et al. (2008) J. Med. Chem. 51:2412-2420), and tarloxotinib (TH-4000), apaziquone (EO9; Oostveen, et al. (1987) Tetrahedron 43:255-262), banoxantrone (AQ4N; Raleigh, et al. (1998) Int. J. Radiat. Oncol. Biol. Phys. 42:763-767), tirapazamine (TPZ; (Shinde, et al. (2009) J. Am. Chem. Soc. 131:14220-14221), SN30000 (CEN-209; Hicks, et al. (2010) Clin. Cancer Res. 16:4946-4957), CH-01 (Cazares-Korner, et al. (2013) ACS Chem. Biol. 8:1451-1459), BCCA621C (Lindquist, et al. (2013) Tumour Microenv. Ther. 1:46-55), O6-alkylguanine DNA alkyltransferase inhibitors (Zhu, et al. (2013) J. Med. Chem. 56:1355-1359, hypoxia-selective EGFR inhibitors (Karnthaler-Benbakka, et al. (2014) Agnew Chem. Int. Ed. Engl. 53:12930-12935), or hypoxia-targeted siRNA (Perche, et al. (2014) Agnew Chem. Int. Ed. Engl. 53:3362-3366).

In a particular embodiment, the molecularly targeted drugs which exploit responses to hypoxia are inhibitors HIF-1α/HIF-1β activity (e.g., HIF-1α inhibitors). Examples of HIF activity inhibitors are provided in Wigerup et al. (Pharmacol. Ther. (2016) 164:152-169). In a particular embodiment, the HIF-1α inhibitor is an inhibitor of mRNA/protein expression (e.g., PI3K inhibitor (e.g., wortmannin, LY94002, GDC-0941, PI-103), mTOR inhibitor (e.g., rapamycin, PP242), aminoflavone, glyceollins, topotecan, PEG-SN38, EZN-2968, 2ME2, ENMD-1198, geldanamycin and analogs, vorinostat, YC-1, PX-478, PX-12, pleurotin, cardiac glycosides, FM19G11, HIF-2α translational inhibitors), an inhibitor of HIF-α/HIF-1β dimerization (e.g., acriflavine, PT2385), inhibitor of DNA binding (e.g., echinomycin, polyamides), and inhibitors of transcriptional activity (e.g., chetomin, bortezomib, amphotericin B, triptolide, AJM290, AW464).

In a particular embodiment, the molecularly targeted drugs which exploit responses to nutrient or oxygen deprivation induced stress are antimetabolites. In a particular embodiment, the antimetabolite agent is 2-mercaptopropionyl glycine disulfide (TTL-315; DuHadaway et al. (2016) Oncotarget 7(7):7372-7380), a disulfide containing compound that blocks cell survival in a manner conditional on glucose deprivation. In a particular embodiment, the agent is a disulfide containing compound listed, without limitation, in U.S. Patent 20140079812. For example, the disulfide containing compound is a di-alkyl disulfides (e.g., disulfides of lower alkyls comprising at least one sulfur atom) or di-aryl disulfides, wherein the members of the disulfide can be the same (symmetrical disulfide) or different (asymmetrical disulfide). In another embodiment, the disulfide containing compounds are disulfides comprising thiamine, such as, without limitation, thiamine disulfide, thiamine propyl disulfide, and thiamine tetrahydrofuryl disulfide. In another embodiment, exemplary disulfide containing compounds include, without limitation, hydroxyethyldisulfide (HEDS; a disulfide of mercaptoethanol (ME)), disulfide of mercaptopropionylglycine (MPG), disulfide of MPG and a lower alkyl, disulfide of MPG and ME, disulfide of mesna (2-sulfanylethanesulfonate), disulfide of MPG and mesna, and disulfide of ME and mesna. In a particular embodiment, the disulfide containing compound is a disulfide of MPG.

In a particular embodiment, the molecularly targeted drugs which exploit responses to nutrient or oxygen deprivation stress are directed against cellular stress signaling pathways. Examples of stress signaling pathways include, without limitation, the unfolded protein response (UPR) and the antioxidant response. An example of an agent directed against the UPR is, without limitation, GSK2656157, a highly selective small molecule inhibitor for the protein kinase R (PKR)- like endoplasmic reticulum kinase (PERK) (Axten et al. (2013) ACS Med Chem Lett 4(10):964-968). PERK mediates a sequential activation of the UPR by phosphorylating eukaryotic initiation factor 2 (eIF2) - thereby stopping global cap dependent translation while activating stress signaling tailored towards alleviating the physiological stress condition (Tabas and David et al (2012) Nat Cell Biol 13(3): 184-190). PERK activation has been extensively showned in various tumors in response to severe hypoxic conditions in its microenvironment and has been shown to be critical for effective survival and proliferation of tumor cells (Bi et al (2005) EMBO J 24 (19): 3470-3481). PERK inhibitors (GSK2656157 as well as GSK2646414) haves been shown to have good oral bioavailability with low to moderate blood clearance in mouse, rat and dog (Axten et al. (2013) ACS Med Chem Lett 4(10):964-968: Atkins et al (2013) Cancer Res. 73(6): 1993-2002; Axten et al (2012) J. Med. Chem. 55(16):7193-207). Treatment with GSK2656157 resulted in delay of various human xenograft tumor growths in mice without significant weight loss andor effect on pancreatic insulin production where PERK is highly expressed (Atkins et al (2013) Cancer Res. 73(6): 1993-2002). Examples of agents directed against the antioxidant response are, without limitation, ZnPPIX, PEG-ZnPPIX, and SMA-ZnPPIX. Hypoxia leads to imbalance of ROS in the tumor microenvironment which in turn activates the master regulator transcription factor NRF2 which regulates genes responsible for eliciting an antioxidant response in tumor cells. An effective inhibitor has not yet been developed against NRF2, however, its primary downstream target heme oxygenase 1 (HO-1) has been successfully targeted for inhibition. HO-1 is a rate-limiting antioxidant enzyme that degrades heme to carbon monoxide (CO), billiverdin and ferrous iron. Along with NRF2, HO-1 expression is regulated by hypoxia activated PERK, and HIF1a and has been shown to be upregulated in various tumors including renal cell carcinoma and prostate cancer, and confers resistance to radiotherapy and photodynamic therapy (Dey, et al. (2015) JCI 125(7):2592-608; Lee, et al. (1997) J. Biol. Chem., 272(9):5375-5381; Berberat, et al. (2005) Clin. Cancer Res., 11(10):3790-3798). An inhibitor of HO-1 enzymatic activity, zinc protoporphyrin IX (ZnPPIX), has been shown to be effective in reducing lymphoma, lung cancer and sarcoma in mice (Jozkowicz, et al. (2007) Antiox Redox Sig 9(12):2099-2117). Improved and more soluble HO-1 inhibitors such as PEG-ZnPPIX and SMA-ZnPPIX haves been shown to have antitumor potential with reduced side effects in mice (Sahoo, et al. (2002) Bioconjug Chem 13(5):1031-1038).

In a particular embodiment, the cancer that may be treated using the compositions and methods of the instant invention include, but are not limited to, prostate cancer, colorectal cancer, pancreatic cancer, cervical cancer, stomach cancer (gastric cancer), endometrial cancer, brain cancer, glioblastoma, liver cancer, bladder cancer, ovarian cancer, testicular cancer, head and neck cancer, throat cancer, skin cancer, melanoma, basal carcinoma, mesothelioma, lymphoma, leukemia, esophageal cancer, breast cancer, rhabdomyosarcoma, sarcoma, lung cancer, small-cell lung carcinoma, non-small-cell carcinoma, adrenal cancer, thyroid cancer, renal cancer, bone cancer, and choriocarcinoma. In a particular embodiment, the cancer forms a tumor. In a particular embodiment, the cancer is lung cancer. In a particular embodiment, the cancer involves pulmonary metastasis. In a particular embodiment, the cancer involves infiltration of IDO-expressing hematopoietic cells in the tumor.

Compositions comprising 1) at least one IDO1 signaling inhibitor and/or 2) at least one anti-vasculogenic/anti-angiogenic agent and/or therapeutic agent which acts by imposing nutrient/oxygen starvation on a target tissue or exploiting nutrient/oxygen starvation in a target tissue are also encompassed by the instant invention. The composition may further comprise at least one carrier (e.g., a pharmaceutically acceptable carrier). In a particular embodiment, the composition comprises 1) at least one IDO1 signaling inhibitor; 2) at least one anti-vasculogenic/anti-angiogenic agent and/or therapeutic agent; and 3) at least one carrier (e.g., a pharmaceutically acceptable carrier). The present invention also encompasses kits comprising a first composition comprising at least one IDO1 signaling inhibitor and a second composition comprising at least one anti-vasculogenic/anti-angiogenic agent and/or therapeutic agent. The first and second compositions may further comprise at least one carrier (e.g., pharmaceutically acceptable carrier). The carriers of the first and second compositions need not be the same.

The agents of the instant invention (e.g., at least one IDO1 signaling inhibitor and at least one anti-vasculogenic/anti-angiogenic agent and/or therapeutic agent which acts by imposing nutrient/oxygen starvation on a target tissue or exploiting nutrient/ oxygen starvation in a target tissue) will generally be administered to a patient as a pharmaceutical preparation. The term "patient" as used herein refers to human or animal subjects. These agents may be employed therapeutically, under the guidance of a physician for the treatment of cancer.

The pharmaceutical preparation comprising the agents of the invention may be conveniently formulated for administration with an acceptable medium such as water, buffered saline, ethanol, polyol (for example, glycerol, propylene glycol, liquid polyethylene glycol and the like), dimethyl sulfoxide (DMSO), oils, detergents, suspending agents or suitable mixtures thereof. The concentration of the agents in the chosen medium may be varied and the medium may be chosen based on the desired route of administration of the pharmaceutical preparation. Except insofar as any conventional media or agent is incompatible with the agents to be administered, its use in the pharmaceutical preparation is contemplated.

The dose and dosage regimen of the agents according to the invention that is suitable for administration to a particular patient may be determined by a physician considering the patient's age, sex, weight, general medical condition, and the specific condition and severity thereof for which the agent is being administered. The physician may also consider the route of administration of the agent, the pharmaceutical carrier with which the agents may be combined, and the agents' biological activity.

Selection of a suitable pharmaceutical preparation depends upon the method of administration chosen. For example, the agents of the invention may be administered by direct injection into any cancerous tissue or into the surrounding area. In this instance, a pharmaceutical preparation comprises the agents dispersed in a medium that is compatible with the cancerous tissue.

Agents may also be administered parenterally by intravenous injection into the blood stream, or by subcutaneous, intramuscular or intraperitoneal injection. Pharmaceutical preparations for parenteral injection are known in the art. If parenteral injection is selected as a method for administering the antibodies, steps must be taken to ensure that sufficient amounts of the molecules reach their target cells to exert a biological effect. The lipophilicity of the agents, or the pharmaceutical preparation in which they are delivered, may be increased so that the molecules can better arrive at their target locations.

Pharmaceutical compositions containing agents of the present invention as the active ingredient in intimate admixture with a pharmaceutical carrier can be prepared according to conventional pharmaceutical compounding techniques. The carrier may take a wide variety of forms depending on the form of preparation desired for administration. In preparing the agent in oral dosage form, any of the usual pharmaceutical media may be employed, such as, for example, water, glycols, oils, alcohols, flavoring agents, preservatives, coloring agents and the like in the case of oral liquid preparations (such as, for example, suspensions, elixirs and solutions); or carriers such as starches, sugars, diluents, granulating agents, lubricants, binders, disintegrating agents and the like in the case of oral solid preparations (such as, for example, powders, capsules and tablets). Because of their ease in administration, tablets and capsules represent the most advantageous oral dosage unit form in which case solid pharmaceutical carriers are obviously employed. If desired, tablets may be sugar-coated or enteric-coated by standard techniques. For parenterals, the carrier will usually comprise sterile water, though other ingredients, for example, to aid solubility or for preservative purposes, may be included. Injectable suspensions may also be prepared, in which case appropriate liquid carriers, suspending agents and the like may be employed.

A pharmaceutical preparation of the invention may be formulated in dosage unit form for ease of administration and uniformity of dosage. Dosage unit form, as used herein, refers to a physically discrete unit of the pharmaceutical preparation appropriate for the patient undergoing treatment. Each dosage should contain a quantity of active ingredient calculated to produce the desired effect in association with the selected pharmaceutical carrier. Procedures for determining the appropriate dosage unit are well known to those skilled in the art. Dosage units may be proportionately increased or decreased based on the weight of the patient. Appropriate concentrations for alleviation of a particular pathological condition may be determined by dosage concentration curve calculations, as known in the art.

In accordance with the present invention, the appropriate dosage unit for the administration of the agents of the invention may be determined by evaluating the toxicity of the agents in animal models. Various concentrations of the agents of the instant invention may be administered to mice with transplanted human tumors, and the minimal and maximal dosages may be determined based on the results of significant reduction of tumor size and side effects as a result of the treatment. Appropriate dosage unit may also be determined by assessing the efficacy of the agents in combination with other standard anti-cancer drugs. The dosage units of the agents may be determined individually or in combination with each anti-cancer treatment according to greater shrinkage and/or reduced growth rate of tumors.

The compositions comprising the agents of the instant invention may be administered at appropriate intervals, for example, at least twice a day or more until the pathological symptoms are reduced or alleviated, after which the dosage may be reduced to a maintenance level. The appropriate interval in a particular case would normally depend on the condition of the patient.

### Definitions

The following definitions are provided to facilitate an understanding of the present invention:
The singular forms "a," "an," and "the" include plural referents unless the context clearly dictates otherwise.

A "therapeutically effective amount" of a compound or a pharmaceutical composition refers to an amount effective to prevent, inhibit, treat, or lessen the symptoms of a particular disorder or disease. For example, "therapeutically effective amount" may refer to an amount sufficient to reduce the cancer burden in a subject.

"Pharmaceutically acceptable" indicates approval by a regulatory agency of the Federal or a state government or listed in the U.S. Pharmacopeia or other generally recognized pharmacopeia for use in animals, and more particularly in humans.

A "carrier" refers to, for example, a diluent, adjuvant, excipient, auxiliary agent or vehicle with which an active agent of the present invention is administered. Pharmaceutically acceptable carriers can be sterile liquids, such as water and oils, including those of petroleum, animal, vegetable or synthetic origin, such as peanut oil, soybean oil, mineral oil, sesame oil and the like. Water or aqueous saline solutions and aqueous dextrose and glycerol solutions are preferably employed as carriers, particularly for injectable solutions. Suitable pharmaceutical carriers are described in "Remington's Pharmaceutical Sciences" by E.W. Martin (Mack Publishing Co., Easton, PA); Gennaro, A. R., Remington: The Science and Practice of Pharmacy, (Lippincott, Williams and Wilkins); Liberman, et al., Eds., Pharmaceutical Dosage Forms, Marcel Decker, New York, N.Y.; and Kibbe, et al., Eds., Handbook of Pharmaceutical Excipients, American Pharmaceutical Association, Washington.

As used herein, the term "prevent" refers to the prophylactic treatment of a subject who is at risk of developing a condition resulting in a decrease in the probability that the subject will develop the condition.

The term "treat" as used herein refers to any type of treatment that imparts a benefit to a patient afflicted with a disease, including improvement in the condition of the patient (e.g., in one or more symptoms), delay in the progression of the condition, etc.

As used herein, the terms "host," "subject," and "patient" refer to any animal, including mammals such as humans.

As used herein, the term "small molecule" refers to a substance or compound that has a relatively low molecular weight (e.g., less than 2,000). Typically, small molecules are organic, but are not proteins, polypeptides, or nucleic acids.

The phrase "small, interfering RNA (siRNA)" refers to a short (typically less than 30 nucleotides long, particularly 12-30 or 20-25 nucleotides in length) double stranded RNA molecule. Typically, the siRNA modulates the expression of a gene to which the siRNA is targeted. Methods of identifying and synthesizing siRNA molecules are known in the art (see, e.g., Ausubel et al. (2006) Current Protocols in Molecular Biology, John Wiley and Sons, Inc). As used herein, the term siRNA may include short hairpin RNA molecules (shRNA). Typically, shRNA molecules consist of short complementary sequences separated by a small loop sequence wherein one of the sequences is complimentary to the gene target. shRNA molecules are typically processed into an siRNA within the cell by endonucleases. Exemplary modifications to siRNA molecules are provided in U.S. Application Publication No. 20050032733. Expression vectors for the expression of siRNA molecules preferably employ a strong promoter which may be constitutive or regulated. Such promoters are well known in the art and include, but are not limited to, RNA polymerase II promoters, the T7 RNA polymerase promoter, and the RNA polymerase III promoters U6 and H1 (see, e.g., Myslinski et al. (2001) Nucl. Acids Res., 29:2502 09).

"Antisense nucleic acid molecules" or "antisense oligonucleotides" include nucleic acid molecules (e.g., single stranded molecules) which are targeted (complementary) to a chosen sequence (e.g., to translation initiation sites and/or splice sites) to inhibit the expression of a protein of interest. Such antisense molecules are typically between about 15 and about 50 nucleotides in length, more particularly between about 15 and about 30 nucleotides, and often span the translational start site of mRNA molecules. Antisense constructs may also be generated which contain the entire sequence of the target nucleic acid molecule in reverse orientation. Antisense oligonucleotides targeted to any known nucleotide sequence can be prepared by oligonucleotide synthesis according to standard methods.

An "antibody" or "antibody molecule" is any immunoglobulin, including antibodies and fragments thereof, that binds to a specific antigen. As used herein, antibody or antibody molecule contemplates intact immunoglobulin molecules, immunologically active portions of an immunoglobulin molecule, and fusions of immunologically active portions of an immunoglobulin molecule.

The antibody may be a naturally occurring antibody or may be a synthetic or modified antibody (e.g., a recombinantly generated antibody; a chimeric antibody; a bispecific antibody; a humanized antibody; a camelid antibody; and the like). The antibody may comprise at least one purification tag. In a particular embodiment, the framework antibody is an antibody fragment. Antibody fragments include, without limitation, immunoglobulin fragments including, without limitation: single domain (Dab; e.g., single variable light or heavy chain domain), Fab, Fab', F(ab')₂, and F(v); and fusions (e.g., via a linker) of these immunoglobulin fragments including, without limitation: scFv, scFv₂, scFv-Fc, minibody, diabody, triabody, and tetrabody. The antibody may also be a protein (e.g., a fusion protein) comprising at least one antibody or antibody fragment.

The antibodies of the instant invention may be further modified. For example, the antibodies may be humanized. In a particular embodiment, the antibodies (or a portion thereof) are inserted into the backbone of an antibody or antibody fragment construct. For example, the variable light domain and/or variable heavy domain of the antibodies of the instant invention may be inserted into another antibody construct. Methods for recombinantly producing antibodies are well-known in the art. Indeed, commercial vectors for certain antibody and antibody fragment constructs are available.

The antibodies of the instant invention may also be conjugated/linked to other components. For example, the antibodies may be operably linked (e.g., covalently linked, optionally, through a linker) to at least one cell penetrating peptide, detectable agent, imaging agent, or contrast agent. The antibodies of the instant invention may also comprise at least one purification tag (e.g., a His-tag). In a particular embodiment, the antibody is conjugated to a cell penetrating peptide.

As used herein, the term "immunologically specific" refers to proteins/polypeptides, particularly antibodies, that bind to one or more epitopes of a protein or compound of interest, but which do not substantially recognize and bind other molecules in a sample containing a mixed population of antigenic biological molecules.

The following example is provided to illustrate various embodiments of the present invention. It is not intended to limit the invention in any way.

### EXAMPLE

### Materials and Methods

### Transgenic Mouse Strains

Congenic *Ido1*^{*-*/*-*} mice on both BALB/c and C57BL/6 strain backgrounds obtained. The development of congenic *Ido2*^{*-*/*-*} mice on the BALB/c strain background has been described (Metz et al. (2014) Int. Immunol., 26:357-367). Congenic *Ifng*^{*-*/*-*} and *Il6*^{*-*/*-*} mouse strains on the BALB/c strain background and WT BALB/c and C57BL/6 strains were acquired from the Jackson Laboratory (Bar Harbor, ME).

### 4T1 Tumor Cell Metastasis

Pulmonary metastasis studies with the 4T1 mouse mammary carcinoma-derived cell line (Aslakson et al. (1992) Cancer Res., 52: 1399-1405) were carried out by injecting 1 × 10⁴ cells in 50 µl serum free medium into the number 4 mammary fatpad to establish an orthotopic tumor which then spontaneously metastasized to the lungs. For visualization of the pulmonary metastasis burden, lungs were inflated with 15% India ink dye, washed, and bleached in Fekete's solution. For immunofluorescence detection of pulmonary metastasis vasculature, lungs were inflated with 50% OCT and frozen in OCT blocks followed by 4 µm sectioning using the CryoJane tape transfer system. Blood vessels within the metastatic nodules were visualized by fluorescence staining with rabbit anti-mouse Caveolin1 polyclonal antibody (Cat. #3238S, Cell Signaling Technology, Boston, MA). For quantitatively evaluating vessel density within the metastatic nodules, multiple fields were acquired per mouse lung on a Zeiss inverted microscope with 40× objective. The pixel density corresponding to the positive Cav1 signal per field was determined in Adobe Photoshop, and these values were then averaged to determine the overall mean value per mouse. For IDO1 inhibitor treatment studies, mice bearing established metastases at 3.5 weeks following 4T1 engraftment were administered 50 mg/kg epacadostat (ChemieTek; Indianapolis, IN) in 100 µl vehicle (3% N,N-dimethylacetamide, 10% 2-hydroxylpropyl-β-cyclodextrin) by oral gavage b.i.d. (twice daily) over 72 hours at which point the animals were euthanized for analysis. Positive control animals were administered a single intraperitoneal (i.p.) injection of 50 mg/kg cyclophosphamide (Baxter; Deefield, IL) in 100 µl sterile saline and euthanized 72 hours later for analysis.

For immunofluorescence microscopy-based visualization of immune cells and IDO1-expressing cells in lung metastases, primary tumor and spleen, resected tissue samples were frozen in OCT blocks followed by 4 µm sectioning using the CryoJane tape transfer system. Immune cells were visualized by fluorescence microscopy following staining as per the manufacturer's instructions with antibodies to the panimmune cell surface marker CD45 conjugated to FITC (Cat. #103122, Biolegend), or to the specific markers for characterizing MDSCs (myeloid derived suppressor cells) Gr1 conjugated to FITC (Cat. #108419, Biolegend) and CD11b conjugated to biotin (Cat. #101204, Biolegend) followed by streptavidin congregated Cy3 (Cat. #405215, Biolegend). Expression of IDO1 in cells was visualized by staining with the IDO1 specific mouse monoclonal antibody 4B7 (Cat. #MABF850, Millipore) followed by a FITC conjugated goat anti-mouse secondary antibody (Cat. #F0257, Sigma) or a Cy3 conjugated goat anti-mouse secondary antibody (Cat. # M30010, Life Technologies) as described (Thomas et al. 20114 J Cell Biochem. 115:391-396). For isolation of the IDO1-expressing immune cell population from 4T1 lung metastases, a single cell suspension was prepared from resected metastatic lung tissue using the gentleMACS Octo Dissociator (Miltenyi Biotec) and Tumor Dissociation Kit (Cat. #130-096-730, Miltenyi Biotec) as per the manufacturer's instructions, and total immune cells were isolated using αCD45 conjugated magnetic beads (Cat. #130-052-301, Miltenyi Biotec) as per the manufacturer's instructions. The IDO1-expressing, Gr1⁺ CD11b⁻cell population was isolated from the total immune cell population by FACS (florescence activated cell sorting) using an FACSAria (Becton Dickinson) cell sorter. The more numerous Gr1⁺ CD11b⁺ cell population, representing conventional MDSCs (myeloid derived suppressor cells), was concurrently isolated as a non-IDO1-expressing control population. For sorting, CD45+ cells were stained with αGr1 antibody conjugated to PerCP and αCD11b antibody conjugated to FITC. For evaluating the effectiveness of the FACS-based cell isolation, cells from both the Gr1⁺ CD11b⁻ and Gr1⁺ CD11b⁺ cell populations were affixed onto slides using a Cytospin 3 and stained with DAPI and with the IDO1 specific mouse monoclonal antibody 4B7 followed by a Cy3 conjugated goat anti-mouse secondary antibody (Cat. # M30010, Life Technologies). For directly assessing the ability of the IDO1-expressing cells to elicit neovascularization *in vivo,* 2 × 10⁶ sorted cells in 250 µL PBS were mixed on ice with 250 µL Matrigel and injected subcutaneously in the dorsal region of a recipient mouse to form a Matrigel plug. At 9 days following implantation, the matrigel plug was excised, photographed, and frozen in an OCT block for cryosectioning. Blood vessels within the Matrigel plug were visualized by fluorescence staining with rabbit anti-mouse caveolin-1 polyclonal antibody (Cat. #3238S, Cell Signaling Technology). For quantitation of vessel density, multiple fields were acquired on a on a Zeiss inverted microscope and analyzed using Adobe Photoshop as described above.

### Statistical Analysis

Graphing and statistical analysis was performed using Prism 6 (GraphPad Software, Inc.). Bar graphs were plotted as means ± SEM with statistical significance determined, as appropriate, by unpaired, 2-tailed Student's t-test or by one-way ANOVA with Tukey's multiple comparison test or Dunnett's multiple comparison test. Significance for Kaplan-Meier survival curves was determined by 2-group log-rank test. P value ranges are indicated as follows: ^{∗∗∗∗}, P < 0.0001; ^{∗∗∗}, P < 0.001; ^{∗∗}, P < 0.01; ^{∗}, P < 0.05; ns, not significant.

### Results

*Ido1*^{*-*/*-*} (*Ido1* nullizygous) mice challenged with orthotopically engrafted 4T1 mammary adenocarcinoma tumors exhibited a marked delay in pulmonary metastasis development relative to their WT (wild type) counterparts (Fig. 1A). When metastases did form in *Ido1*^{*-*/*-*} hosts, they were observed to have a lower vascular density than those formed in WT animals (Fig. 1B), the significance of which was confirmed by quantitative analysis (Fig. 1C; Δ = 2.6-fold, P < 0.01). This finding indicates that neovascularization of the pulmonary 4T1 metastases is impaired in mice lacking IDO1.

The genetic data suggest that pharmacologic inhibitors of IDO1 enzymatic activity may have therapeutic potential for reducing pathological neovascularization. Epacadostat was administered by oral gavage over a 72 hour period to mice with established 4T1 pulmonary metastases. Epacodostat (INCB024360) is a specific, small molecule inhibitor of IDO1 (Liu et al. (2010) Blood 115:3520-3530). Treatment with epacadostat resulted in a significant reduction in metastatic tumor neovascularization relative to vehicle alone (Δ = 3.2-fold, P < 0.01) that was comparable to the effect of the positive control compound cyclophosphamide (Ibe et al. (2001) J. Exp. Med., 194:1549-1559) (Figs. 2A and 2B). These results confirm that blocking the enzymatic activity of IDO1 through pharmacologic inhibition is sufficient to interfere with the ability of IDO1 to support neovascularization.

The inflammatory cytokine IFNγ is a major inducer of IDO1. There is substantial evidence that IFNγ is critical for effective anti-tumor immunity (Beatty et al. (2001) Immunol. Res., 24:201-210) and IFNγ-elicited reduction of the tumor neovasculature may be a mechanism of action (Qin et al. (2000) Immunity 12:677-686; Qin et al. (2003) Cancer Res., 63:4095-4100). Here, the impact of the loss of both IFNγ and IDO1 in the host animal on neovascularization in 4T1 pulmonary metastases was examined. Metastases that formed in *Ido1*^{*-*/*-*} hosts exhibited a significantly lower vascular density relative to those that formed in the wild type controls, while the concomitant loss of both IFNγ and IDO1 in double knockout mice completely negated the effect on 4T1 metastasis neovascularization of IDO1 loss alone (Figs. 3A and 3B). The loss of IDO1 provides a significant survival benefit to mice challenged orthotopically with 4T1 tumors due to reduced pulmonary metastasis burden (Smith et al. (2012) Cancer Discov., 2:722-735). In this context, the concomitant loss of IFNγ together with IDO1 in double knockout mice negated both the reduction in pulmonary metastasis burden and the survival benefit observed with the loss of IDO1 alone (Figs. 3C and 3D). These data demonstrate a striking correspondence between the countervailing effects of IDO1 and IFNγ on neovascularization and metastasis survival, indicating that the antagonistic effect that IDO1 has on the anti-neovascular activity of IFNγ is directly linked to its pro-tumorigenic role in this metastasis model.

The loss of IDO1 is associated with attenuated IL6 induction in the 4T1 metastasis model and metastasis susceptibility can be restored by provision of exogenous IL6 (Smith et al. (2012) Cancer Discov., 2:722-735). IL6 loss affected neovascularization in 4T1 lung metastases, with the metastatic tumors obtained from *Il6*^{*-*/*-*} animals exhibiting a significantly reduced vascular density relative to the wild type controls (Figs. 4A and 4B). Again, the concomitant loss of IFNγ abrogated the reduction in vascular density observed with the loss of IL6 alone (Figs. 4A and 4B). Loss of IL6 was likewise associated with a clear reduction in pulmonary metastasis burden and increased survival benefit for mice challenged with orthotopic 4T1 tumors, a benefit that was lost with the concomitant loss of IFNγ (Figs. 4C and 4D). The corresponding similarity of the effects of IL6 loss and IDO1 loss on neovascularization and metastasis susceptibility is consistent with IL6 acting as an important downstream mediator of IDO's effects on these processes.

Elevation of IDO1 enzymatic activity corresponds with 4T1 metastasis outgrowth in the lungs (Smith et al. (2012) Cancer Discov., 2:722-735). To determine the cell type in which IDO1 is expressed, an IDO1 specific antibody was used to detect the presence of IDO1 in tissue samples by fluorescence microscopy. Immunofluorescence staining of metastatic lung nodules revealed that IDO1 is expressed in tumor infiltrating immune cells and not in the tumor cells themselves (Figure 5A). IDO1⁺ immune cells were concentrated in the lung metastases but not in the primary 4T1 tumor nor in the spleen suggesting either that a specific IDO1-expressing subtype localizes to 4T1 lung metastases or that IDO1 is specifically elevated in this particular microenvironment (Figure 5A). Further evaluation revealed that the IDO1⁺ cells in the 4T1 lung metastases are also positive for the cell surface marker Gr1 (Figure 5B). Gr1 is one of the defining markers in mice for an immune cell population referred to as MDSCs (myeloid derived suppressor cells). The other marker most commonly used to identify MDSCs is CD11b, but unexpectedly IDO1 expression did not colocalize with this marker. Thus, IDO1 is expressed in a population of immune cells related to but distinct from classical MDSCs. Unlike the Gr1⁺ CD11b⁺ MDSCs, which have been intensely studied due to their immune suppressive activity, very little information has been reported on the Gr1⁺ CD11b⁻ cell population and there is no previous association of these cells with IDO1 expression.

In order to functionally assess the role of the IDO1-expressing Gr1⁺ CD11b⁻cell population in supporting neovascularization, these cells were isolated from 4T1 lung metastases. Total CD45⁺ immune cells were sorted by flow cytometry to isolate the Gr1⁺ CD11b⁻ subset (Figure 6A). The much more prevalent Gr1⁺ CD11b⁺ cell population, representing conventional MDSCs, was isolated concurrently as an IDO1 nonexpressing comparator cell population. Fluorescence microscopy analysis of these sorted cell populations confirmed that IDO1 positivity was restricted to the Gr1⁺ CD11b⁻ cells while CD11b positivity was restricted to the Gr1⁺ CD11b⁺ cells (Figure 6B). To assess the ability of specific cell populations to promote neovascularization *in vivo*, FACS isolated cells mixed with Matrigel were embedded under the skin to form a subcutaneous plug. After 9 days the resected plug was assessed for infiltrating neovascularization both visually and by immunofluorescence staining of serial sections. Plugs prepared with Gr1⁺ CD11b⁻ cells showed evidence of pronounced neovascularization compared to the negative control plugs with no added cells (Figure 6C). Quantitatively, this amounted to a 190-fold increase in vascular density associated with the presence of the Gr1⁺ CD11b⁻ cells (Figure 6D), confirming the sufficiency of this isolated cell population to promote neovascularization. In contrast, only marginal evidence of neovascularization was observed in plugs prepared with Gr1⁺ CD11b⁺ cells (Figure 6C, 6D), indicating that this activity is not indiscriminately associated with immature myeloid cells but rather is specific to the CD11b⁻ subtype. To evaluate whether the expression of IDO1 in Gr1⁺ CD11b⁺ cells contributes to their ability promote neovascularization, FACS isolated cells were prepared from 4T1 lung metastases established in *Ido1*^{*-*/*-*} mice. In contrast to cells obtained from WT mice, the *Ido1*^{*-*/*-*} Gr1⁺ CD11b⁻ cells were ineffectual in promoting neovascularization similar to their Gr1⁺ CD11b⁺ counterparts (Figure 6C, 6D), indicating that IDO1 is essential to the ability of these cells to promote neovascularization.

The data provided herein establishes a clear biological role for IDO1 in supporting pathologic neovascularization and shows that IDO1 promotes this outcome through its integration at the regulatory interface between two competing inflammatory cytokines, IFNγ and IL6. Neovascularization was substantially reduced with the loss of IDO1, and this effect was completely reversed by the concomitant loss of IFNγ, a primary inducer of IDO1. The loss of IL6, known to exhibit IDO1 dependent expression, likewise resulted in reduced neovascularization that was also determined to be IFNγ dependent. Direct tumor relevance was corroborated in an orthotopic 4T1 pulmonary metastasis model in which corresponding effects on neovascularization, metastatic tumor burden, and survival were observed.

Although the tolerogenic properties of IDO1 have been extensively explored, the possibility that IDO1 might also impact neovascularization has not, to this point, been generally recognized nor clearly established as biologically relevant. Notably, human xenograft tumors engineered to overexpress exogenous IDO1 exhibit increased vascular density (Nonaka et al. (2011) Int. J. Oncol., 38:113-120; Li et al. (2006) J. Invest. Dermatol., 126:128-136). Normal pulmonary vascularization is also diminished in *Ido1* nullizygous mice (Smith et al. (2012) Cancer Discov., 2:722-735). The present data demonstrates the importance of endogenous IDO1 for supporting neovascularization and presents a clear correlation between IDO1-dependent neovascularization and the outgrowth of lung metastases. The present data also provides mechanistic evidence to explain the basis for this effect by linking IDO1 to regulatory interactions with the inflammatory cytokines IFNγ and IL6.

In addition to being expressed in immune cells, such as dendritic cells and macrophages, IDO1 expression in endothelial cells has also been identified (Blaschitz, et al. (2011) PLoS One 6:e21774). Biochemically, by catabolizing tryptophan, IDO1 can signal through two distinct metabolic pathways, one responding to downstream tryptophan catabolites and the other to the depletion of tryptophan itself. Both mechanisms have been linked to the positive regulation of IL6 (Dinatale et al. (2010) Toxicol. Sci., 115:89-97; Liu et al. (2014) Mol. Cell. Biol., 34:428-438) via kynurenine signaling through AHR (aryl hydrocarbon receptor) or amino acid depletion signaling through GCN2 (general nonderepressible 2).

In addition to establishing IDO1's role in supporting neovascularization, the data provided herein shows that the use of a pharmacologic inhibitor is an effective intervention strategy against neovascularization. In the 4T1 metastasis model, administration of epacadostat to mice harboring established, vascularized metastases resulted in a reduced level of neovascularization in response to treatment. Thus, rather than simply reiterating IDO1's role in supporting neovascular development, this result indicates that treatment with an IDO1 inhibitor effectively degrades established neovascular networks. The antineovascular effect of cyclophosphamide is IFNγ-dependent (Ibe et al. (2001) J. Exp. Med., 194:1549-1559), which, if not directly linked, also suggests a mechanistic convergence with IDO1.

IDO1 small molecule inhibitors are currently being evaluated in clinical trials for a variety of cancers based on the supposition that they will help to enable an effective immune response directed against the tumor. This study indicates, however, that neovascularization is also involved. The clear interconnection established between the effects of IDO1, IFNγ and IL6 loss on neovascularization and metastasis survival is consistent with other indications of the importance of the anti-neovascular effect that IFNγ exerts against tumors (Qin et al. (2000) Immunity 12:677-686; Qin et al. (2003) Cancer Res., 63:4095-4100). Accordingly, the impact on the tumor vasculature should additionally be considered when assessing clinical results obtained with IDO1 inhibitors, particularly in the context of pulmonary metastases.

An emergent concept in the field of angiogenesis is that normalizing pathologic tumor vasculature will enhance the efficacy of chemo- and radiotherapies. Synergistic responses obtained with combinations of IDO1 signaling inhibitors and chemotherapeutic agents in different mouse tumor models has been observed (Hou et al. (2007) Cancer Res., 67:792-801; Muller et al. (2005) Nat. Med., 11:312-319). Additionally, it was noted in a clinical study that the IDO1 pathway inhibitor, indoximod, appeared to sensitize tumors to salvage chemotherapy (Soliman et al. (2013) J. Clin. Oncol., 31(suppl): abstr 3069), which would be consistent with the vascular normalization improving chemotherapy responses. IDO1 inhibition may also enhance the IFNγ-dependent, anti-angiogenic effect elicited by certain chemotherapeutic drugs such as cyclophosphamide, which has been reported to produce immune-dependent rejection of large, vascularized tumors mediated by the destruction of tumor blood vessels in a manner that requires IFNγ receptor expression on normal host cells (Ibe et al. (2001) J. Exp. Med. 194:1549-1559). By eliminating the negative feedback constraint imposed by IDO1 on the anti-angiogenic effect of IFNγ, IDO1 inhibitors can enhance this aspect of the therapeutic response to cyclophosphamide and other chemotherapeutic drugs that have also been identified as having anti-angiogenic activity, such as taxanes (Bocci et al. (2002) Cancer Res., 62:6938-6943).

## Claims

1. A combination for use in the treatment, inhibition, and/or prevention of cancer in a subject, the combination comprising:
(a) at least one inhibitor of indoleamine 2,3-dioxygenase-1 (IDO1), wherein the IDO1 inhibitor is 1-methyl-tryptophan, 1-methyl-D-tryptophan (indoximod), or a racemic mixture containing 1-methyl-D-tryptophan; a small molecule or antibody that binds and inhibits IDO1; or a nucleic acid molecule that inhibits IDO1 expression; and
(b) at least one protein kinase R (PKR)-like endoplasmic reticulum kinase (PERK) inhibitor; or at least one hypoxia-activated prodrug or bioreductive drug.

2. The combination for use of claim 1, wherein the combination comprises a small molecule and the small molecule is epacadostat or navoximod.

3. The combination for use of claim 1, wherein the combination comprises a nucleic acid molecule and the nucleic acid molecule that inhibits IDO1 expression is an siRNA, shRNA, or an antisense oligonucleotide.

4. The combination for use of any of claims 1 to 3, wherein combination comprises a PERK inhibitor and the PERK inhibitor is GSK2656157 or GSK2606414.

5. The combination for use of any of claims 1 to 4, wherein the combination comprises a IDO1 inhibitor and a PERK inhibitor, and wherein the IDO1 inhibitor and PERK inhibitor are in a single composition comprising at least one pharmaceutically acceptable carrier or wherein the IDO1 inhibitor and the PERK inhibitor are in separate compositions each comprising at least one pharmaceutically acceptable carrier.

6. The combination for use of any of claims 1 to 4, wherein the combination comprises a IDO1 inhibitor and a PERK inhibitor and wherein the IDO1 inhibitor and the PERK inhibitor are formulated for administration sequentially or concurrently, optionally wherein the IDO1 inhibitor and the PERK inhibitor are formulated for administration close enough in time such that the IDO1 inhibitor and the PERK inhibitor are capable of acting synergistically.

7. The combination for use of any of claims 1 to 3, wherein the combination comprises a hypoxia-activated prodrug or a bioreductive drug and said hypoxia-activated prodrug or bioreductive drug is PR-104, evofosfamide, or tarloxotinib.

8. The combination for use according to any of claims 1 to 3 or 7, wherein the combination comprises a IDO1 inhibitor and a hypoxia-activated prodrug or bioreductive drug and wherein the IDO1 inhibitor and the hypoxia-activated prodrug or bioreductive drug are in a single composition comprising at least one pharmaceutically acceptable carrier or wherein the IDO1 inhibitor and the hypoxia-activated prodrug or bioreductive drug are in separate compositions each comprising at least one pharmaceutically acceptable carrier.

9. The combination for use according to any one of claims 1 to 3 or 7, wherein the combination comprises a IDO1 inhibitor and a hypoxia-activated prodrug or bioreductive drug and the IDO1 inhibitor and the hypoxia-activated prodrug or bioreductive drug are formulated for administration sequentially or concurrently, optionally wherein the IDO1 inhibitor and the hypoxia-activated prodrug or bioreductive drug are formulated for administion close enough in time such that the IDO1 inhibitor and the hypoxia-activated prodrug or bioreductive drug are capable of acting synergistically.

10. The combination for use of any one of claims 1 to 9, wherein said cancer is lung cancer, optionally wherein said lung cancer comprises pulmonary metastasis.

11. A pharmaceutical composition comprising:
at least one inhibitor of indoleamine 2,3-dioxygenase-1 (IDO1), wherein the IDO1 inhibitor is 1-methyl-tryptophan, 1-methyl-D-tryptophan (indoximod), or a racemic mixture containing 1-methyl-D-tryptophan; a small molecule or antibody that binds and inhibits IDO1; or a nucleic acid molecule that inhibits IDO1 expression;
at least one PERK inhibitor or at least one hypoxia-activated prodrug or bioreductive drug; and
at least one pharmaceutically acceptable carrier.

12. The pharmaceutical composition of claim 11, wherein the composition comprises a small molecule and the small molecule is epacadostat or navoximod.

13. The pharmaceutical composition of claim 12, wherein the composition comprises a nucleic acid molecule and the nucleic acid molecule that inhibits IDO1 expression is an siRNA, shRNA, or an antisense oligonucleotide.

14. The pharmaceutical composition of any one of claims 11 to 13, wherein the composition comprises a PERK inhibitor and the PERK inhibitor is GSK2656157 or GSK2606414.

15. The pharmaceutical composition of any one of claims 11 to 14, wherein the composition comprises a hypoxia-activated prodrug or bioreductive drug and said hypoxia-activated prodrug or bioreductive drug is PR-104, evofosfamide, or tarloxotinib.
